# EUROPEAN PATENT APPLICATION

(11) **EP 0 679 888 A2**
(43) Date of publication of application: **02.11.1995**
(21) Application number: 95106191.0
(22) Date of filing: 26.04.1995
(51) Int. Cl.: G01N 33/48, G01N 33/60, G01N 33/68, C12Q 1/02, G01N 33/50

(54) **Method for determining capability of imparting growth activity**

(30) Priority: 28.04.1994 JP 114387/94
(71) Applicant: Kureha Chemical Industry Co., Ltd., Tokyo 103 (JP)
(72) Inventor: Oguchi, Yoshiharu, Nerika-ku, Tokyo 179 (JP); Matsunaga, Kenichi, Tokorozawa-shi, Sautana 359 (JP)
(74) Representative: Minderop, Ralph H., Dr. rer. nat.

(57) **Abstract**

A method for determining capability of a sample to impart growth activity to an in vitro cultured cell line sensitive to an immunoregulatory factor, comprising cultivating cultured cells from said cell line in the presence of said sample, and evaluating said capability of said sample in comparison with capability obtained in a control cultivation of said cultured cells in the absence of said sample is disclosed. The method can be used to assay an immunological activity of said sample, and then diagnose an immune disease.

## Description

### BACKGROUND OF THE INVENTION

### 1. Field of the Invention

The present invention relates to a method of measuring the capability of a serum sample or the like to impart growth activity to cultured cells sensitive to immunoregulatory factors. By determining the capability of a sample to impart growth activity to the cultured cells by means of the method of the present invention, it is possible to determine the immunological activity of patients and use the results for diagnosis of diseases of the immune system.

### 2. Description of the Related Art

Typical examples of indicators clinically used in the conventional methods to show the decline in the immunological activity of patients suffering from diseases include a skin test for delayed-type hypersensitivity such as a skin reaction against purified protein derivative (PPD) and against 2,4-dinitrochlorobenzene (DNCB) as *in vivo* tests; and the count of leukocytes in peripheral blood, the count of subsets of lymphocytes in peripheral blood by monoclonal antibodies, the measurement of the blastogenesis rate of lymphocytes in peripheral blood in response to phytohemagglutinin (PHA), the measurement of the content of immunosuppressive acidic protein (IAP) in serum, and the measurement of the stimulation index (SI) (measurement of suppressive activity in serum on mouse spleen cell blastogenesis reaction), or the like as *ex vivo* tests.

However, the immunological host defense mechanism is complicated and it is extremely difficult to assess the immunological activity of patients only by one parameter. Particularly, in the case of malignant diseases such as cancer, the immune state of the host is closely related to its clinical progress and prognosis. However, no immunological indicator capable of accurately grasping the immunological activity and prognosis of the patient was known. Therefore, development of such an indicator was strongly desired.

### SUMMARY OF THE INVENTION

The inventors of the present invention engaged in intensive research to solve the above problem, and as a result discovered that the growth factor dependent growth reaction of certain types of cultured cell lines belonging to lymphoid cell lineage is strongly affected by the immunoregulatory factors in the patient's humor, and that by examining the changes of the growth reaction rate, it is possible to determine the immunological activity in the patient's humor and further to use these results for diagnosis of immune diseases of patients.

Accordingly, the object of the present invention is to provide a means for determining the immunological activity in patient's humor and further use the results therefrom for diagnosis of immune diseases of the patients, by cultivating a growth factor dependent cultured lymphoid cell line in the presence of the patient's humor and examining the changes of the growth reaction rate of the cultured cell line.

Other objects and advantages will become clear from the following description.

Therefore, the present invention relates to a method for determining capability of a sample to impart growth activity to an in vitro cultured cell line sensitive to an immunoregulatory factor, comprising cultivating cultured cells from said cell line in the presence of said sample, and evaluating said capability of said sample in comparison with capability obtained in a control cultivation of said cultured cells in the absence of said sample.

The present invention also relates to a method for assay of an immunological activity of a sample, comprising cultivating cultured cells from an in vitro cultured cell line sensitive to an immunoregulatory factor in the presence of said sample, and evaluating capability of said sample to impart growth activity to said cultured cells in comparison with capability obtained in a control cultivation of said cultured cells in the absence of said sample.

Further, the present invention relates to a method for diagnosing an immune disease, comprising cultivating cultured cells from an in vitro cultured cell line sensitive to an immunoregulatory factor in the presence of a sample, evaluating capability of said sample to impart growth activity to said cultured cells in comparison with capability obtained in a control cultivation of said cultured cells in the absence of said sample, and determining an immunological activity of said sample.

### BRIEF DESCRIPTION OF THE DRAWINGS

Figure 1 is a graph illustrating the suppression of growth of CTLL-2 cells by the serum of a cancer patient.

Figure 2 is a graph illustrating the suppression of growth of KOA-1 cells by the serum of a cancer patient.

### DESCRIPTION OF THE PREFERRED EMBODIMENTS

In many diseases accompanied with changes in the immunological functions, the patient's humor contains various substances affecting the immunological functions, that is, immunoregulatory factors. For example, in cancer accompanied with a reduction of the immunological functions, the activity to inhibit the immunological functions is recognized in the serum of the patient. The immune response, however, includes numerous reactions and the sensitivity with respect to immunoreguratory factors varies with different immunoreactions. The inventors of the present invention discovered that there exist cultured cells (in particular, cell lines from T-cells) which are highly sensitive to immunoregulatory factors, such as an immunosuppressive factors, participating in the various reactions involved in these immune responses, and that a growth reaction (in particular, a cytokine dependent growth reaction) of the cultured cells sensitive to immunoregulatory factors are considerably influenced in the presence of the immunoregulatory factors, and that by determining the changes in the growth rate, it is possible to evaluate the action of the immunoregulatory factors.

The sample used in the method of the present invention is not limited so long as it is a sample to be examined with respect to the immunological activity, but for example is a sample of a biological fluid, in particular, blood, serum, plasma, marrow fluid, urine, ascitic fluid, or the like of human or other animals. These samples may be used as they are or in some cases diluted with physiological saline, a buffer, or a culture medium.

In the method of the present invention, cells from cultured cell lines sensitive to immunoregulatory factors are cultivated in the presence of the sample and the capability of the sample to impart growth activity to the cells sensitive to immunoregulatory factors is measured. It is preferable to use cells from cytokine-dependent cultured cell lines sensitive to immunoregulatory factors and to carry out the growth reaction of said cells in the presence of cytokine and a sample, because it is possible to evaluate the influence of the sample on the growth reaction with a high sensitivity. The term "capability of imparting growth activity" used herein means the capability of the immunoregulatory factors contained in the sample to exert an influence upon the growth of the cultured cells sensitive to immunoregulatory factors, and specifically means the action of suppressing growth and the action of accelerating growth.

The cultured cells or cells from cell lines sensitive to immunoregulatory factors which may be used in the present invention are not particularly limited so long as they are cells influenced in growth reaction by the immunoregulatory factors contained in the sample, but cultured cells which are sensitive to immunoregulatory factors and exhibit a cytokine dependent growth are preferable in that they reflect the immune response in the living body and are highly sensitive to immunoregulatory factors. Many cultured cells exhibit a cytokine-dependent growth are known [Tanpakushitsu, Kakusan, Koso (Proteins, Nucleic Acids, Enzymes), 35, 875, 1990] . Of these cells, cultured cells which are sensitive to immunoregulatory factors and exhibit an interleukin 2 (IL-2) dependent growth are preferable in that interleukin 2 plays an extremely important role in many immune reactions in the living body. For example, it is possible to use CTLL-2 or HT-2 from mouse, or ILT-Mat or KOA-1 from human, or the like. The cultured cells are easily available from various microorganism depository institutions or the like. For example, mouse-derived CTLL-2 (ATCC TIB214) (RCB637) is available from the American Type Culture Collection (ATCC) or the Riken (Rikagaku Kenkyusho) Cell Bank. Further, it is possible to newly establish cultured cells having such properties by means of known methods.

A growing step of the cultured cells in the present invention can be carried out under the cultivating conditions ordinarily and generally used in general *in vitro* culture, except that the sample (or a control sample or the like) is added. When a cytokine dependent cultured cells are used, a cytokine or a substance having same activity is added in ordinary amounts. For example, when CTLL-2 is used, the cell can be cultivated under the culture conditions generally used while adding human or mouse IL-2 or a substance having same activity, although the composition of the culture medium or the like can be somewhat modified.

The amount of the sample added is determined by preliminary experiments performed in advance, and within the range in which dependence on the sample added is obtained.

As the cells used in the present invention, cells subcultured for 2 to 10 days are preferable, because they exhibit a high growth activity and can bring about stable results. Cells subcultured for 2 to 5 days are particularly preferable, because the growth activity thereof is especially high. When the assay is carried out in the present invention, it is preferable to adjust the concentration of immunoregulatory factor sensitive cultured cells used so as not to cause excessive growth and thus, reduction of growth during the cultivation. For example, in the case of CTLL-2 cells, the concentration is preferably maintained at less than about 1 x 10⁶ cells/ml during the cultivation. On the other hand, if the number of cells at the time of the determination is small, the determination sensitivity falls. Therefore, it is necessary to adjust the cell concentration at the time of starting the cultivation so as to reach a certain range. The cultivation term before the measurement is preferably 2 to 4 days because the significant difference can be observed. For example, when CTLL-2 cells are cultivated for 2 days before the determination, the initial cell concentration should be around 1 x 10⁵ cells/ml. When CTLL-2 cells are used, a change in the cell number varying with that in the amount of IL-2 added is observed. Therefore, it is necessary to adjust the amount of IL-2 added in accordance with the purpose of the determination. In general, adjustment is preferably made to the extent that sufficient growth of CTLL-2 is observed and the IL-2 does not become in excess to cause a reduction in the sensitivity of determination.

After the cultivation, the ability of the sample to impart growth activity, that is, the effect on the rate of cell growth, is determined. Specifically, it is possible to use the count of the cell number, measurement of the amount of ³H-thymidine incorporated into cells, or an MTT assay [an assay of 3-(4,5-dimethylthiazol-2-yl)-2,5-diphenyl-tetrazolium-bromide] or the like. It is preferable to measure the amount of ³H-thymidine incorporated or perform an MTT assay, because of excellent measurement efficiency.

It is possible to use various improved methods of the MTT assay, for example, methods using XTT [2,3-bis(2-methoxy-4-nitro-5-sulfophenyl)-5-[(phenylamino)carbonyl]-2H-tetrazolium-hydroxide] or WST-1 [sodium salt of 4-[3-(4-iodophenyl-2-(4-nitrophenyl)-2H-5-tetrazolio]-1,3-benzene-disulfonate], or the like.

In the present invention, a uniform group of the cultured cells sensitive to immunoregulatory factors are used, and therefore a high sensitivity is obtained in the measurement of the amount of the ³H-thymidine uptake and the MTT assay, or the like. In the MTT assay, A₅₇₀-A₆₃₀ are generally measured to evaluate the results. Although the reactivity varies more or less from a measurement to a measurement, it is possible to reduce the effects of the above fluctuations by simultaneously measuring a corresponding positive control in all runs and make corrections.

A method for immunodiagnosis similar to the present invention, namely, a method using mouse spleen cells was reported (Hattori, T. et al; Jpn. J. Surg., 20, 127 - 136, 1990). However, the method of the present invention is superior in the following points compared with the above conventional method:
(1) The sensitivity to immunoregulatory factors is higher, and therefore, accuracy and sensitivity in determination are better.
(2) Cultured cells are used, and cell population with a uniform responsibility are obtained. Therefore, reproducibility is better.
(3) Animals per se are not used, and assay can be performed without facilities for raising animals.
Therefore, the present invention can be widely used.

According to the method of the present invention, it is possible to measure the immunological activity with a high sensitivity and reproducibility, and the immunological activity of patients suffering from immuno defective diseases such as cancer, infectious diseases, auto immune disorders, or the like can be determined. Thus, the present invention can be used for diagnosis of immune diseases.

### Examples

The present invention will now be further illustrated by, but is by no means limited to, the following Examples.

### Example 1

### (1) Subculture of CTLL-2 Cells

CTLL-2 (RCB637: distributed from Riken Cell Bank) was cultivated, using a culture medium prepared by adding, to RPMI 1640 medium, 10% bovine fetal serum, 5 x 10⁻⁵M 2-mercaptoethanol, 50 µg/ml gentamycin, and 20 units/ml of mouse rIL-2 (Genzyme Co.), in a CO₂ incubator (5% CO₂, 95% air) at 37°C. The subculture was maintained at an interval of 3 to 4 days. The number of the cells at the time of subculturing was adjusted to keep the cell number lower than 1 x 10⁶ cells/ml.

### (2) Measurement of Effect of Human Serum on Growth of CTLL-2 Cells

The sera from cancer patients (24 samples), healthy subjects (21 samples) or bovine fetal serum were diluted with a culture medium (prepared by adding to RPMI 1640 medium, 5 x 10⁻⁵M 2-mercaptoethanol and 50 µg/ml of gentamycin: hereinafter referred to as the "culture medium") to obtain various dilutions. The resulting dilutions were placed in the wells of a 96-well flat bottom microplate, a CTLL-2 cell suspension and further mouse rIL-2 were added, and incubation was performed in a CO₂ incubator at 37°C. The cell concentration was adjusted to 1 x 10⁻⁵ cells/ml, the concentration of mouse rIL-2 was adjusted to 10 units/ml, and the amount of the liquid in each well was adjusted to 100 µl.

To prepare the CTLL-2 cells used above, cells from the maintenance subculture of the above item (1) were cultivated in a concentration of 1 x 10⁻⁵ cells/ml, harvested at the second day, centrifuged and washed 3 times with the culture medium.

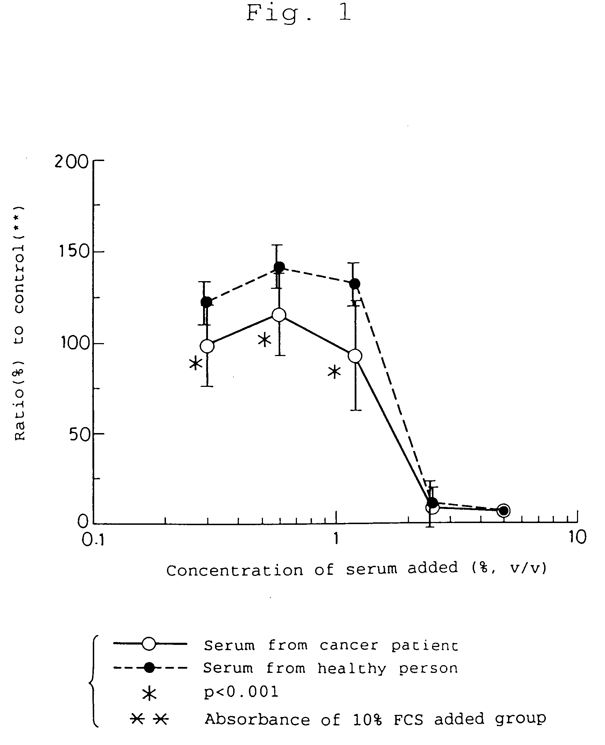

After cultivating the cells for 44 hours, 15 µl of a solution prepared by dissolving MTT [3-(4,5-dimethylthiazol-2-yl)-2,5-diphenyl-tetrazolium-bromide] in a phosphate buffered saline to a concentration of 5 mg/ml was added to each of the wells and the cells were cultivated further for 4 hours. After the cultivation was finished, 200 µl of 40 mM isopropanol acidified with hydrochloric acid was added to each well. Yielded formazan was dissolved by pipetting. The absorbance of each well (A₅₇₀-A₆₃₀) was measured by a spectrophotometer (Microplate Reader Model 3550: BIO-RAD). The absorbance of a standard group using dilutions prepared by adding bovine fetal serum at a final concentration of 10% was calculated as 100 and the absorbances of the test groups were calculated as percentages to the above value 100. The results are shown in Fig. 1. The solid line shows the results for serum from cancer patients and the dashed line shows the results for serum from healthy subjects in Fig. 1. Further, the transversal axis of Fig. 1 is a logarithmic scale.

As clear from Fig. 1, significant suppression of growth of CTLL-2 was observed in the serum from cancer patients, as compared with that from healthy subjects, in the range of 0.3 to 1.25% of the serum added. In other words, it is possible to evaluate the immunosuppressive activity in the serum of cancer patients by the method of the present invention.

### Example 2

### (1) Subculture of KOA-1 Cells

IL-2 dependent cell strain KOA-1 (FERM BP-4631) established from human peripheral lymphocytes was cultivated, using a culture medium prepared by adding, to RPMI 1640 medium, 10% bovine fetal serum, 5 x 10⁻⁵M 2-mercaptoethanol, 50 µg/ml gentamycin, and 300 units/ml of human rIL-2 (Genzyme Co.), in a CO₂ incubator (5% CO₂, 95% air) at 37°C. The subculture was maintained at an interval of 3 to 7 days. The number of the cell at the time of subculturing was adjusted to keep the cell number in the range of 1 to 15 x 10⁵ cells/ml all the time.

The above-mentioned KOA-1, i.e. human IL-2 dependent lymphocyte KOA-1, was deposited on April 8, 1994 in National Institute of Bioscience and Human-Technology Agency of Industrial Science and Technology of the Japanese Ministry of International Trade and Industry (address: 1-3, Higashi 1-chome, Tsukuba-shi, Ibaraki 305, Japan) under the deposition No. FERM BP-4631.

### (2) Measurement of Effect of Human Serum on Growth of KOA-1 Cells.

The sera from cancer patients (sample A) which had been examined in advance in Example 1 and was found to exhibit an immunosuppressive activity, and the sera from healthy subjects (sample B) were diluted with the culture medium to obtain various dilutions. The resulting dilutions were placed in the wells of a 96-well flat bottom microplate, a KOA-1 cell suspension and further human rIL-2 were added, and incubation was performed in a CO₂ incubator at 37°C. The cell concentration was adjusted to 2 x 10⁵ cells/ml, the concentration of human rIL-2 was adjusted to 300 units/ml, and the amount of the liquid in each well was adjusted to 100 µl.

To prepare the KOA-1 cells used above, cells from the maintenance subculture of the above item (2) in Example 2 were cultivated, centrifuged and washed with the culture medium.

After cultivating the cells for 3 days, 25 µl of a solution of [6-³H]thymidine (Amersham Co.) diluted with the culture medium to a concentration of 740 KBq/ml was added to each of the wells and the cells were cultivated further for 1 day. After the cultivation was finished, a cell harvester (Cambridge Technology Co.) was used to harvest the cells on a glass fiber filter by an ordinary method and the radioactivity was measured by a liquid scintillation counter containing a scintillator (NEN Co.: Aquasol II).

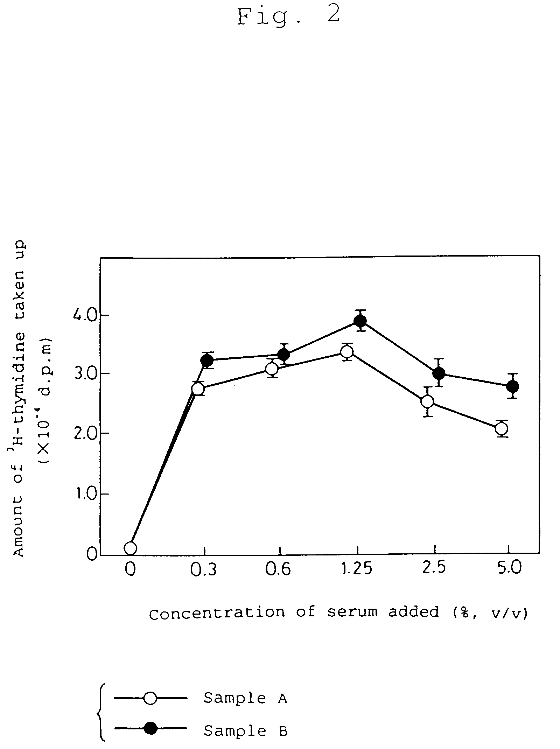

The results are shown in Fig. 2. As clear from Fig. 2, a significant difference was observed between the sample A (○ mark in Fig. 2) and the sample B (● mark in Fig. 2) in the range between 0.3 and 5.0% of the serum added. That is, it is also possible to use this embodiment to evaluate the immunosuppressive activity in the serum of cancer patients in the same way as the first embodiment.

As above, the present invention was explained with reference to particular embodiments, but modifications and improvements obvious to those skilled in the art are included in the scope of the present invention.

## Claims

1. A method for determining capability of a sample to impart growth activity to an in vitro cultured cell line sensitive to an immunoregulatory factor, characterized by cultivating cultured cells from said cell line in the presence of said sample, and evaluating said capability of said sample in comparison with capability obtained in a control cultivation of said cultured cells in the absence of said sample.

2. A method according to claim 1, wherein said in vitro cultured cell line sensitive to an immunoregulatory factor is a cultured cell line exhibiting a cytokine dependent growth activity.

3. A method according to claim 2, wherein said cultured cell line exhibiting a cytokine dependent growth activity is a cultured cell line exhibiting an interleukin 2 dependent growth activity.

4. A method according to claim 3, wherein said cultured cell line exhibiting an interleukin 2 dependent growth activity is CTLL-2 from mouse.

5. A method according to claim 3, wherein said cultured cell line exhibiting an interleukin 2 dependent growth activity is KOA-1 from human.

6. A method according to any one of claims 1 to 5, wherein said capability to impart growth activity is determined by counting cell number, measuring an amount of radioactive isotopes incorporated, or an enzymatic reaction.

7. A method for assay of an immunological activity of a sample, characterized by cultivating cultured cells from an in vitro cultured cell line sensitive to an immunoregulatory factor in the presence of said sample, and evaluating capability of said sample to impart growth activity to said cultured cells in comparison with capability obtained in a control cultivation of said cultured cells in the absence of said sample.

8. A method according to claim 7, wherein said sample is a biological fluid.

9. A method according to claim 8, wherein said biological fluid is serum.

10. A method for diagnosing an immune disease, characterized by cultivating cultured cells from an in vitro cultured cell line sensitive to an immunoregulatory factor in the presence of a sample, evaluating capability of said sample to impart growth activity to said cultured cells in comparison with capability obtained in a control cultivation of said cultured cells in the absence of said sample, and determining an immunological activity of said sample.
